# EUROPEAN PATENT SPECIFICATION

(11) **EP 3 618 767 B1**
(45) Date of publication and mention of the grant of the patent: **23.04.2025**
(21) Application number: 18726634.1
(22) Date of filing: 02.05.2018
(51) Int. Cl.: A61F 2/24

(54) **EXPANDABLE SHEATH TECHNOLOGIES**
TECHNOLOGIEN FÜR EXPANDIERBARE SCHLEUSEN
TECHNOLOGIES DE GAINE EXTENSIBLE

(30) Priority: 04.05.2017 US 201762501167 P
(43) Date of publication of application: 11.03.2020
(73) Proprietor: St. Jude Medical, Cardiology Division, Inc., St. Paul, MN 55117 (US)
(72) Inventor: NESS, Peter J., Minneapolis, Minnesota 55417 (US); RHODE, Nathan B., Arden Hills, Minnesota 55112 (US); FRASCONE, Martin P., Maplewood, Minnesota 55119 (US); TURCIOS-CARRERA, Eber J., Minneapolis, Minnesota 55406 (US); WITSCHEN, Michael T., Monticello, Minnesota 55362 (US); LAKE, Will A., Wauwatosa, Wisconsin 53226 (US); NELSON-CHEESEMAN, Brittany, Minneapolis, Minnesota 55406 (US)
(74) Representative: Valea AB
(86) International application number: PCT/US2018/030633
(87) International publication number: WO 2018/204486

(56) References cited:
- WO-A1-2017/040774
- US-A1- 2013 178 711
- US-A1- 2016 095 622

## Description

### Cross-Reference to Related Applications

This application claims the benefit of the filing date of U.S. Provisional Patent Application No. 62/501,167, filed May 4, 2017.

### Background of the Disclosure

The present disclosure relates to systems and devices for minimally invasive delivery of medical devices, such as collapsible and expandable heart valves, into a patient.

Various minimally invasive medical procedures involve obtaining access to a patient's vasculature with the aid of an introducer sheath. The introducer sheath may provide a structure through which another medical device may be passed into and through the vasculature. Procedures such as transcatheter aortic valve replacement ("TAVR"), endovascular aneurysm repair ("EVAR"), and thoracic endovascular aortic repair ("TEVAR") each may involve obtaining access to a patient's vasculature through an artery of the patient, such as the femoral artery. The sheath of an introducer may be passed into the artery, and then a delivery device holding a collapsible and expandable medical device, such as a stent with a prosthetic valve (for TAVR) or a stent with a graft (for EVAR and TEVAR), may be advanced to the site of interest via the introducer sheath while the medical device is in a collapsed condition. When the delivery device reaches the site of interest, the medical device may be released therefrom and expanded into place at the desired site. The delivery device and introducer sheath may then be removed from the patient.

**In** these and other procedures, particularly those involving large bore access, shear and longitudinal forces applied to the patient's vasculature by introducers may cause undesirable trauma to the vasculature. As used herein, the term large bore access may refer to procedures that employ catheters having an external size of at least 14 French (4.667 mm diameter). To reduce trauma to the patient's vasculature, it may be desirable to employ an introducer with an outer diameter that is small relative to the inner diameter of the vessel in which the introducer is to be positioned. However, the inner diameter of the introducer must be sufficiently large to accommodate the outer diameter of the device or devices which will pass through the introducer. These countervailing parameters have led to the use of expandable introducer sheaths, which may be inserted into and removed from the vasculature at a constricted diameter, but which may expand to an expanded diameter - either passively or actively - as a secondary device with a diameter larger than the first constricted diameter is passed through the introducer sheath. US2013178711 A1 discloses an introducer for providing access to a surgical site comprising a sheath including a coil having a plurality of turns spiraling about the longitudinal axis, wherein the coil has a reduced diameter configuration and an expanded diameter configuration.

### Brief Summary

The present invention is directed to an introducer for providing access to a surgical site as defined in claim 1. The dependent claims define further aspects of the invention.

According to a first aspect of the disclosure, an introducer for providing access to a surgical site in a patient includes a sheath. The sheath extends from a proximal portion to a distal portion along a longitudinal axis, the sheath including a coil having a plurality of turns spiraling about the longitudinal axis. The coil has a reduced diameter configuration in which portions of the coil forming a multiplicity of the turns are spaced a first distance from the longitudinal axis in a radial direction orthogonal to the longitudinal axis, and an expanded diameter configuration in which the portions of the coil forming the multiplicity of the turns are spaced a second distance from the longitudinal axis in the radial direction. The second distance is greater than the first distance.

According to a second aspect of the disclosure, an introducer for providing access to a surgical site in a patient includes a sheath. The sheath extends from a proximal portion to a distal portion along a longitudinal axis, the sheath including a frame having a spine extending from the proximal portion of the sheath to the distal portion of the sheath in a longitudinal direction parallel to the longitudinal axis. The frame includes a plurality of first ribs spaced apart from one another in the longitudinal direction and a plurality of second ribs spaced apart from one another in the longitudinal direction. Each first rib has a first end coupled to a first edge of the spine extending in the longitudinal direction and a free end remote from the first end. Each second rib has a first end coupled to a second edge of the spine extending in the longitudinal direction opposite the first edge and a free end remote from the first end. The plurality of first ribs and the plurality of second ribs extend in a circumferential direction at least partially around the longitudinal axis. The frame has a reduced diameter configuration in which the free end of a first rib is spaced a first distance from the free end of an adjacent second rib, and an enlarged diameter configuration in which the free end of the first rib is spaced a second distance from the free end of the adjacent second rib. The second distance is greater than the first distance.

According to a third aspect of the disclosure, an introducer for providing access to a surgical site in a patient includes a sheath. The sheath extends from a proximal portion to a distal portion along a longitudinal axis, the sheath including a frame having first and second supports extending from the proximal portion of the sheath to the distal portion of the sheath in a longitudinal direction parallel to the longitudinal axis. The frame includes a plurality of flexible rings coupled to the first and second supports, the flexible rings extending circumferentially around the longitudinal axis. The frame has an expanded diameter configuration in which the plurality of flexible rings have a circular shape and a plane defined by the circular shape is substantially orthogonal to the longitudinal axis, and a reduced diameter configuration in which the plurality of flexible rings have an elliptical shape and a plane defined by the elliptical shape is non-orthogonal to the longitudinal axis.

According to a fourth aspect of the disclosure, an introducer for providing access to a surgical site in a patient includes a sheath. The sheath extends from a proximal portion to a distal portion along a longitudinal axis, the sheath including a frame having a plurality of struts forming a plurality of cells having a repeating pattern, the frame extending circumferentially around the longitudinal axis. The frame has an expanded diameter configuration in which the cells have a first geometric shape, the frame has a first interior diameter, and the sheath has a first distance in a longitudinal direction parallel to the longitudinal axis between the proximal portion of the sheath and the distal portion of the sheath. The frame also has a reduced diameter configuration in which the cells have a second geometric shape, the frame has a second interior diameter smaller than the first interior diameter, and the sheath has a second distance in the longitudinal direction between the proximal portion of the sheath and the distal portion of the sheath. The first geometric shape is different than the second geometric shape and the first distance is different than the second distance.

### Brief Description of the Drawings

Fig. 1 is a side view of a delivery device for a prosthetic heart valve assembled to an introducer.
Fig. 2A is a top plan view of a portion of the operating handle of the delivery device of Fig. 1, shown with a partial longitudinal cross-section of the distal portion of a transfemoral catheter assembly.
Fig. 2B is a side view of the operating handle of Fig. 2A.
Fig. 3 is a perspective view of the introducer of Fig. 1.
Fig. 4A is a cut-away view of a portion of an expandable sheath of one embodiment of an introducer similar to that shown in Fig. 3.
Fig. 4B is a highly schematic partial view of a coil of the sheath of Fig. 4A in a reduced diameter configuration.
Fig. 4C is a highly schematic partial view of the coil of the sheath of Fig. 4A in an expanded diameter configuration.
Fig. 4D is a cross-section of a coil similar to the coil of Figs. 4B-C.
Fig. 4E is an enlarged cross-sectional view through one turn of the coil of Fig. 4D.
Fig. 5A is a perspective view of the distal end of an expandable sheath of another embodiment of an introducer similar to that shown in Fig. 3.
Fig. 5B is a side view of a portion of the sheath of Fig. 5A.
Fig. 5C is a schematic transverse cross-sectional view of the sheath of Fig. 5A in a reduced diameter configuration.
Fig. 5D is a schematic transverse cross-sectional view of the sheath of Fig. 5A in an expanded diameter configuration.
Fig. 6A is a perspective view of the distal end of an expandable sheath of another embodiment of an introducer similar to that shown in Fig. 3, shown in an expanded diameter configuration.
Fig. 6B is a perspective view of the distal end of the sheath of Fig. 6A, shown in a reduced diameter configuration.
Figs. 6C-D are schematic side views of a portion of the sheath of Fig. 6A in the expanded diameter configuration and the reduced diameter configuration, respectively.
Figs. 6E-F illustrate the concept that the orthogonal projection of an ellipse may form a circle.
Fig. 7A is a perspective view of yet another embodiment of an introducer, with an enlarged view of the frame of the expandable sheath in an expanded diameter configuration.
Fig. 7B is a perspective view of the introducer of Fig. 7A, with an enlarged view of the frame of the sheath in a reduced diameter configuration.
Figs. 8A-B schematically illustrate an auxetic structure in collapsed and expanded conditions, respectively.
Fig. 8C is a perspective view of still another embodiment of an introducer, with an enlarged view of the frame of the expandable sheath incorporating an auxetic structure, the frame being illustrated in a reduced diameter configuration.

### Detailed Description

As used herein, the term "proximal," when used in connection with an introducer and/or delivery device, refers to the end of the device closer to the user of the device when the device is being used as intended. On the other hand, the term "distal," when used in connection with an introducer and/or delivery device, refers to the end of the device farther away from the user. The term "longitudinal direction" generally refers to a direction along or parallel to the longitudinal axis of a structure. The term "radial direction" generally refers to a direction extending radially toward or away from the longitudinal axis of the relevant structure. The term "circumferential direction" generally refers to a direction that encircles the longitudinal axis of the relevant structure. In the figures, like numbers refer to like or identical parts. As used herein, the terms "substantially," "generally," "approximately," and "about" are intended to mean that slight deviations from absolute are included within the scope of the term so modified. When ranges of values are described herein, those ranges are intended to include sub-ranges. For example, a recited range of 1 to 10 includes 2, 5, 7, and other single values, as well as all sub-ranges within the range, such as 2 to 6, 3 to 9, 4 to 5, and others.

Although the introducers described herein are described in conjunction with their use in the implantation of collapsible prosthetic heart valves, it should be understood that these introducers may be used in connection with other suitable procedures. To provide additional context for the use of the introducers disclosed herein in connection with the implantation of prosthetic heart valves, additional information regarding such valves is provided below.

Prosthetic heart valves that are collapsible to a relatively small circumferential size can be delivered into a patient less invasively than valves that are not collapsible. For example, a collapsible valve may be delivered into a patient via a tube-like delivery device such as a catheter, a trocar, a laparoscopic instrument, or the like. This collapsibility can avoid the need for a more invasive procedure such as full open-chest, open-heart surgery.

Collapsible prosthetic heart valves typically take the form of a valve structure mounted on a stent. There are two types of stents on which the valve structures are ordinarily mounted: a self-expanding stent and a balloon-expandable stent. To place such valves into a delivery device and ultimately into a patient, the valve must first be collapsed or crimped to reduce its circumferential size.

Generally, when implanting a prosthetic heart valve into a patient, an introducer sheath is first introduced into the desired blood vessel or other anatomy. This may occur after a guidewire and one or more dilators are introduced into the patient. The introducer sheath may be advanced a distance into the vasculature. Once the introducer sheath is in place, it is used as a conduit to pass other devices, as necessary, from outside the patient into the vasculature. For example, a delivery device containing the prosthetic heart valve in a collapsed condition may be passed through the introducer sheath so that the prosthetic heart valve may be deployed and implanted at the desired native heart valve annulus.

When the collapsed prosthetic valve has reached the desired implant site in the patient (e.g., at or near the annulus of the native heart valve that is to be replaced by the prosthetic valve), the prosthetic valve can be deployed or released from the delivery apparatus and re-expanded to full operating size. For balloon-expandable valves, this generally involves releasing the entire valve, assuring its proper location, and then expanding a balloon positioned within the valve stent. For self-expanding valves, on the other hand, the stent automatically expands as the sheath covering the valve is withdrawn.

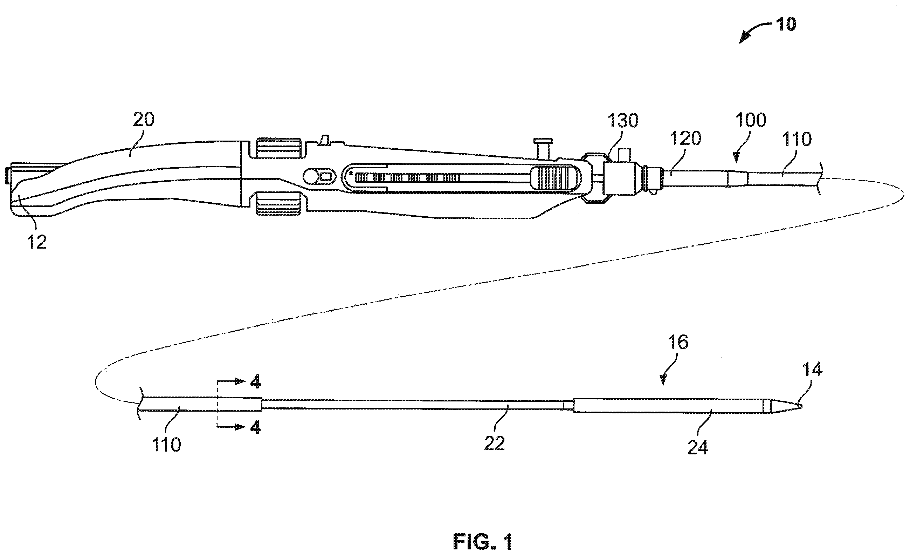

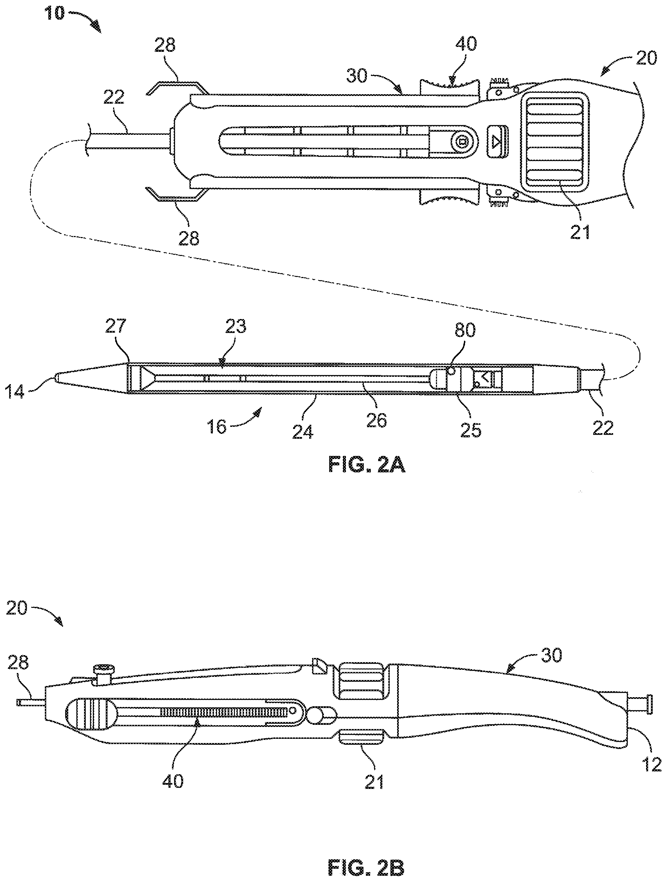

Fig. 1 shows a prosthetic heart valve delivery device 10 assembled to an introducer 100, while Figs. 2A-B show the general features of delivery device 10. Generally, delivery device 10 includes a catheter assembly 16 for delivering the heart valve to, and deploying the heart valve at, a target location, and an operating handle 20 for controlling the deployment of the valve from the catheter assembly. Catheter assembly 16 includes an outer shaft 22 extending from operating handle 20 and a distal sheath 24 connected to the distal end of the outer shaft. A compartment 23 defined around an inner shaft 26 in catheter assembly 16 is configured to receive a prosthetic heart valve (not shown), which is held in a collapsed condition when distal sheath 24 covers the compartment.

Delivery device 10 extends from a proximal end 12 at the proximal end of operating handle 20 to an atraumatic tip at the distal end of catheter assembly 16. The inner shaft 26 of catheter assembly 16 may extend through operating handle 20 to the atraumatic tip 14 of delivery device 10, and may include a retainer 25 affixed thereto at a spaced distance from the atraumatic tip. Retainer 25 may have recesses 80 therein that are adapted to receive corresponding retention members of the prosthetic valve to thereby hold the valve in place in compartment 23. Distal sheath 24 surrounds inner shaft 26 and is slideable relative to inner shaft 26 such that it can selectively cover or uncover compartment 23. The distal end 27 of distal sheath 24 abuts atraumatic tip 14 when the distal sheath fully covers compartment 23, and is spaced apart from the atraumatic tip when the compartment is at least partially uncovered.

Operating handle 20 is adapted to control the deployment of the prosthetic valve from compartment 23 by permitting a user to selectively slide outer shaft 22 proximally or distally relative to inner shaft 26, thereby respectively uncovering or covering compartment 23 with distal sheath 24. The proximal end of inner shaft 26 may be connected in a substantially fixed relationship to the outer housing 30 of operating handle 20, and the proximal end of outer shaft 22 may be affixed to a carriage assembly 40 that is slideable along a longitudinal axis of the handle housing, such that a user can selectively slide the outer shaft relative to the inner shaft by sliding the carriage assembly relative to the handle housing. For example, a user may rotate a deployment actuator 21 in operating handle 20 to move carriage assembly 40 proximally, thus moving outer shaft 22 and distal sheath 24 proximally to uncover a prosthetic heart valve positioned within compartment 23. As distal sheath 24 begins to uncover the prosthetic heart valve, the prosthetic heart valve begins to expand to an expanded condition so that it may be fixed within the native heart valve annulus of interest.

In one embodiment, the distal sheath 24 of delivery device 10 may have a size of between about 18 French and about 19 French. Outer shaft 22 may have a size of between about 12 French and about 13 French.

The distal end of handle 20 may include a latching element configured to latch or otherwise lock onto a complementary latching element of introducer 100. Although shown in Figs. 2A-B as a pair of hooks 28, any other suitable latching mechanism, including snaps, knobs, or similar mechanisms may be used, as described in greater detail below. One handle that may be suitable for use is described in greater detail in U.S. Patent Publication No. 2013/0297011, the disclosure of which is hereby incorporated by reference herein.

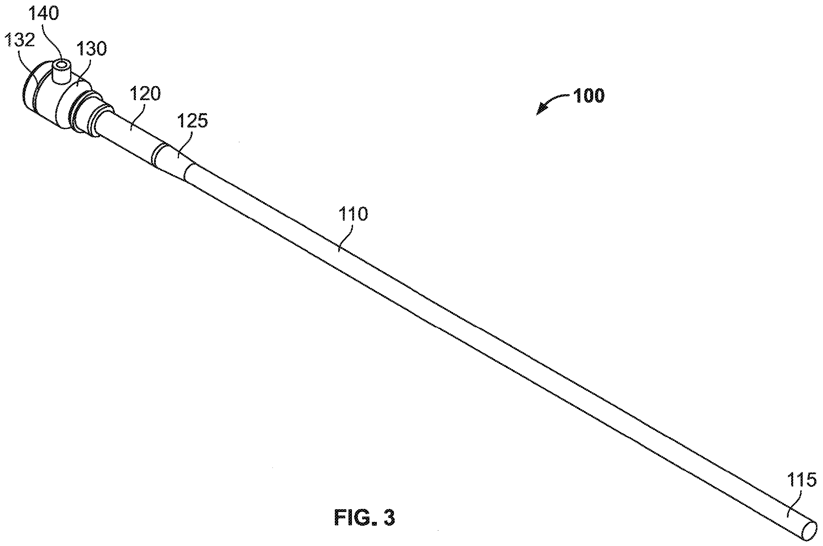

Now referring to Fig. 3, introducer 100 generally includes a hollow distal sheath 110 connected to a hollow proximal sheath 120. A housing 130 connected to proximal sheath 120 may include additional components including, for example, a flush port 140 and one or more seals or hemostasis valves (not illustrated) to prevent blood from flowing proximally out of introducer 100. Housing 130 may also include a latching element 132 for mating with latching element 28 of handle 20. For example, latching element 132 may be a groove, snap, aperture, or other mechanism configured to couple with latching element 28. In one embodiment, proximal sheath 120 may have diameter size of approximately 19 French and include a tapered portion 125 at its distal end. The distal end 115 of distal sheath 110 may also taper to a reduced diameter in some embodiments. Preferably, distal sheath 110 is expandable from a first diameter to a larger second diameter. In this and other embodiments described herein, it is preferable that the outer diameter of sheath 110 in the reduced diameter configuration is about 4 mm (12 French), while the inside diameter of the sheath is about 6.67 mm (20 French) in the expanded diameter configuration. Various mechanisms which may provide for the expandability of distal sheath 110 are described below.

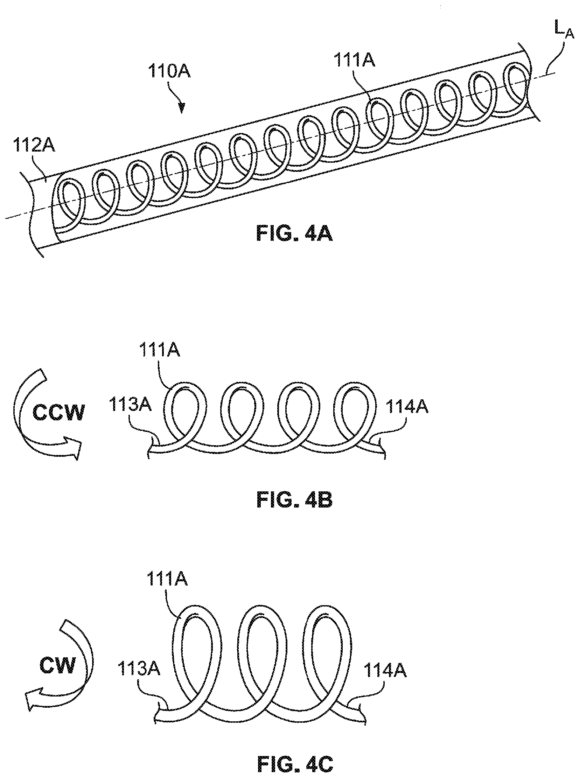

Fig. 4A is a cutaway view of a first embodiment of an expandable distal sheath 110A which may form a part of introducer 100. Sheath 110A may include a wire or coil 111A that forms a continuous spiral extending the entire length or substantially the entire length of sheath 110A. Coil 111A may spiral around a longitudinal axis L_{A} extending in a proximal-to-distal direction along sheath 110A so that a substantially hollow center is formed, with each point of the coil being substantially equally radially spaced from the longitudinal axis. In order to maintain a fluid-tight seal between the space inside coil 111A and the space outside the coil, an expandable outer layer 112A may cover the coil. In other embodiments, an extrusion, impregnation, or other processes may be used such that layer 112A forms a substantially cylindrical tube with coil 111A extending at least partially inside the walls and/or lumen of that tube. Although various materials may be used to form coil 111A, polytetrafluoroethylene ("PTFE") may be particularly suitable because of its low coefficient of friction. Layer 112A is preferable formed of a highly elastic material to allow for expansion and contraction of coil 111A, described in greater detail below. In one embodiment, layer 112A is formed from a polyimide material with a hydrophilic coating. However, it should be understood that other materials than those listed may be suitable for performing the same or similar functions, and the particular materials do not limit the scope of the invention unless explicitly stated otherwise.

In order to increase the diameter of coil 111A, a user may begin to unwind the coil. For example, coil 111A is shown having a first relatively small diameter in Fig. 4B, the coil extending between a first end 113A and a second end 114A opposite the first end and having loops that wind in the counter-clockwise direction from the first end to the second end. A user may grasp first end 113A and begin to unwind coil 111A, for example by rotating the first end in the direction labeled counterclockwise (CCW) in Fig. 4B. If second end 114A is held stationary during counterclockwise rotation of first end 113A, the overall length of coil 111A along the longitudinal axis L_{A} may decrease as the diameter of the coil increases, as shown in Fig. 4C. On the other hand, in order to reduce the diameter of coil 111A, a user may grasp first end 113A and begin to wind coil 111A, for example by rotating the first end in the direction labeled clockwise (CW) in Fig. 4C. If second end 114A is held stationary during clockwise rotation of first end 113A, the overall length of coil 111A. along the longitudinal axis L_{A} increases as the diameter of the coil decreases, as shown in Fig. 4B. Although not shown, a relatively rigid bar, wire, or other structure may be coupled to the second end 114A of coil 111A and extend proximally to a location near first end 113A, so that the second end may be held substantially stationary while the first end is rotated. Preferably, as coil 111A either increases or decreases in diameter, each individual turn of the coil increases or decreases substantially uniformly, so that the diameter of the coil is substantially constant along the length of sheath 110A. In other embodiments, second end 114A of coil 111A may be rigidly fixed to a distal end of layer 112A. In such an embodiment, layer 112A may be held at its proximal end, which may assist in keeping second end 114A of coil 111A. relatively stationary while first end 113A is wound or unwound.

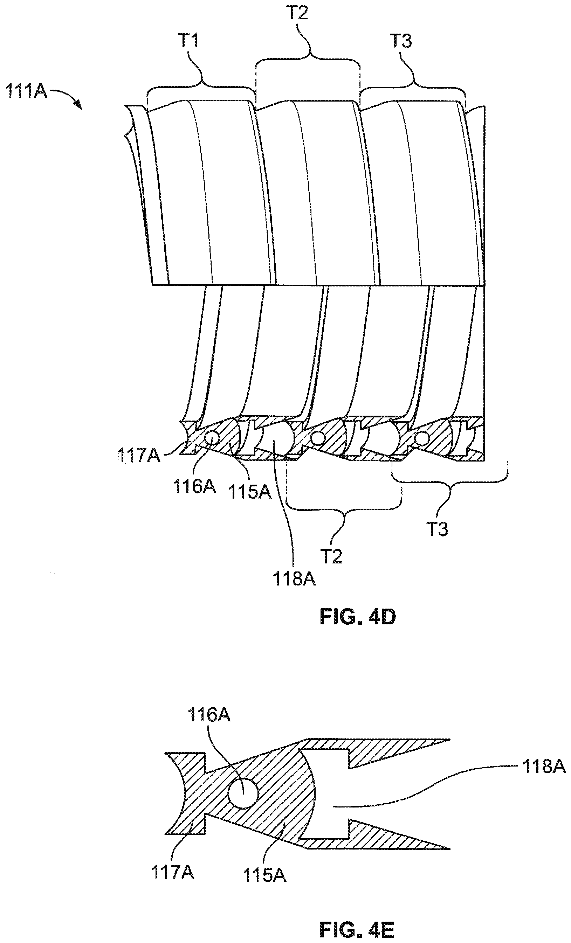

Although in the embodiment shown in Figs. 4A-C, coil 111A is depicted as a wire, the coil may take other forms. For example, a side view of coil 111A in partial cross-section is shown in Fig. 4D, with three turns T1-T3 of the coil illustrated, although it should be understood that the coil may include more than three turns. Each turn T1-T3 may be defined by a complete revolution of coil 111A, with each turn being continuous with an adjacent turn. As best shown in Fig. 4E, each turn T1-T3 may include a body 115A having an aperture 116A. Body 115A may taper in a first direction and transition into a flange 117A that is wider than the narrowest portion of the taper. On the opposite side of aperture 116A, body 115A may have sidewalls that extend to form two pointed prongs that define recess 118A between them. As shown in Fig. 4D, the flange 117A of one turn may passively sit within a portion of the recess 118A of a next adjacent turn. A pull-wire (not illustrated) may be positioned within aperture 116A and extend through each turn of coil 111A, with one end of the pull-wire fixed to the distal end of the coil and the opposite end of the pull-wire extending proximally of the proximal end of the coil so that it is accessible by the user. Pulling the pull-wire proximally, while the distal end of the pull wire is fixed to the distal end of coil 111A, causes the diameter of the coil to increase as the distalmost end of the coil retracts towards the proximal end of the coil as the turns of the coil unwind.

In some embodiments, coil 111A may be formed of a shape-memory material shape-set to the expanded diameter so that, in the absence of applied force, the coil tends to unwind to the expanded diameter. In these embodiments, sheath 110A (including coil 111A and layer 112A) may be kept in the reduced diameter configuration by being positioned within a covering sheath (not shown) having a non-expandable or minimally expandable internal diameter that is substantially equal to the outer diameter of the coil when the coil is in the reduced diameter configuration. Sheath 110A may be inserted into the vasculature of the patient at the access site while maintained in the reduced diameter configuration by the covering sheath. The insertion of sheath 110A in the reduced diameter configuration may avoid trauma to the vasculature that might otherwise occur with a larger diameter sheath. Once fully inserted, the covering sheath may be retracted, exposing sheath 110A and allowing it to expand to the expanded diameter configuration. A proximal end of the covering sheath may include a longitudinal slit or other feature to help ensure the covering sheath could be temporarily pulled proximally over larger portions of other structures (such as housing 130) positioned proximal to the covering sheath. Delivery device 10 (or another suitable secondary device) may be inserted through sheath 110A while the sheath is in the expanded diameter configuration. The procedure may be continued, for example by implanting a prosthetic heart valve, and delivery device 10 may be removed from the patient while sheath 110A remains in the expanded diameter configuration. Prior to removing sheath 110A from the patient, it may be transitioned back to the reduced diameter configuration, for example via active manipulation of coil 111A as described above. In other embodiments, after removal of delivery device 10 from the patient, the covering sheath may be advanced back over sheath 110A to force coil 111A to transition back to the reduced diameter configuration. Then, the covering sheath and sheath 110A may be withdrawn from the patient while sheath 110A remains within the covering sheath.

In still other embodiments, coil 111A may tend to revert to the reduced diameter configuration in the absence of applied forces, for example by shape-setting the coil to the reduced diameter configuration. In such embodiments, sheath 110A may be inserted and removed from the patient without a covering sheath or any other mechanism for maintaining coil 111A in the reduced diameter configuration. Once sheath 110A is in the desired position within the patient, delivery device 10 may be passed through the sheath, with the relatively large size of the delivery device forcing local portions of coil 111A to increase in diameter as the delivery device passes through the coil. Alternately, the first end 113A of coil 111A may be rotated in the desired direction while the second end 114A of the coil remains substantially stationary, forcing the entire length of the coil to transition to the expanded diameter configuration. The expanded diameter configuration of coil 111A may be maintained while delivery device 10 is advanced through sheath 110A. The expanded diameter configuration of coil 111A could be maintained by keeping first end 113A and second end 114A of the coil substantially fixed with respect to one another, which could be accomplished manually or with the assistance of a lever, actuator, or other suitable structure. Once the prosthetic heart valve has been implanted, delivery device 10 may be removed from sheath 110A and the patient, at which point the first end 113A of coil 111A may be released, enabling the coil to revert to the reduced diameter configuration and the sheath to be removed from the patient. In the embodiments in which coil 111A reverts to the reduced diameter configuration in the absence of applied forces, the covering sheath described in connection with the embodiments above may be omitted.

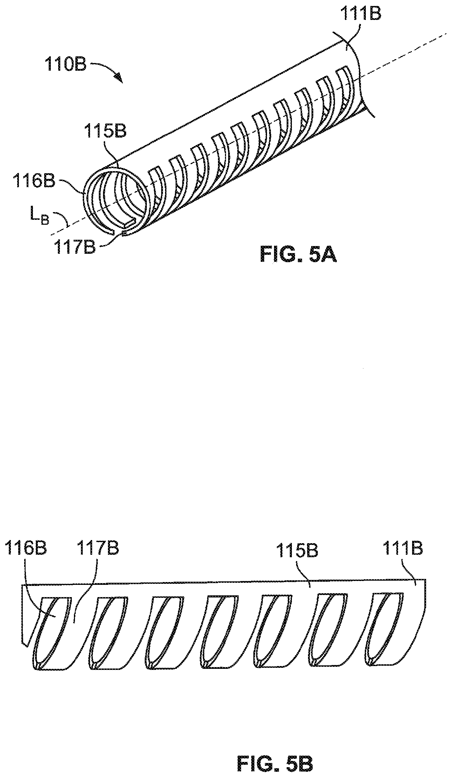

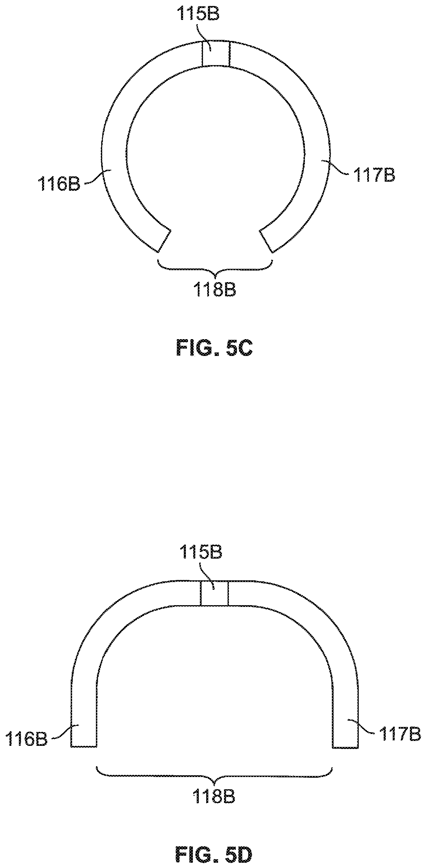

Fig. 5A is a perspective view of a portion of another embodiment of an expandable distal sheath 110B which may form a part of introducer 100. Sheath 110B may include a frame 111B with a spine 115B extending the entire length of the sheath. Spine 115B may be substantially rectangular but with a radius of curvature, such that the spine forms a portion of a cylinder having a central longitudinal axis coextensive with the longitudinal axis L_{B} of sheath 110B. A plurality of first ribs 116B may be integral with spine 115B and extend from a first lateral edge of the spine, curving about longitudinal axis L_{B}. A plurality of second ribs 117B may be integral with spine 115B and extend from a second lateral edge of the spine, also curving about longitudinal axis L_{B}. The plurality of first ribs 116B may be spaced apart from one another in the proximal-to-distal direction, and the plurality of second ribs 117B may be similarly spaced. In one embodiment, each first rib 116B may be at the same longitudinal position non spine 115B as a corresponding second rib 117B, such that the two ribs and the corresponding portion of the spine collectively form a portion of a circle or cylinder, as best seen in Fig. 5C. Each first rib 116B and each second rib 117B may terminate at a free end so that a gap 118B is formed between the adjacent free ends of each corresponding first and second rib.

In the illustrated embodiment, frame 111B forms a substantially hollow center, with each point of the frame being substantially equally radially spaced from longitudinal axis L_{B} in the absence of forces applied to the frame. In order to maintain a fluid-tight seal between the space inside frame 111B and the space outside the frame, an expandable outer layer of material, similar to layer 112A of sheath 110A, may cover the exterior of the frame. For example, a thin cylindrical covering may extend around the exterior of frame 111B, including around the spacing between adjacent first ribs 116B, around the spacing between adjacent second ribs 117B, and around gaps 118B. The material covering the exterior surfaces of frame 111B is preferably thin and able to expand and contract as the diameter of the frame changes, while maintaining a fluid tight seal between the inside of the frame and the outside of the frame. Similar to the material forming layer 112A of sheath 110A, the material covering the exterior of frame 111B may be a polyimide material having a hydrophilic coating to help reduce friction. The interior surface of frame 111B may be similarly covered with a polyimide material with a hydrophilic coating, such that the frame is effectively encapsulated by the coating or covering material. In other words, the layers of material, alone or in combination with frame 111B, may provide for a substantially cylindrical, fluid tight structure that may locally change diameters, as described below. However, it should be understood that other materials than those explicitly listed above may be suitable as well.

In one embodiment, frame 111B may be formed of a shape-memory material such as Nitinol and shape-set so that, in the absence of applied forces, spine 115B and corresponding ribs 116B, 117B form a continuous smooth curve in the shape of a portion of a cylinder. It should be understood that other materials, including flexible metals and/or polymers that may or may not be shape-memory materials, may be suitable for forming frame 111B. Sheath 110B (including frame 111B and the covering layer or layers) may be inserted into the vasculature of the patient at the access site while maintained in the cylindrical shape. This may be referred to as the reduced diameter configuration. Once sheath 110B is fully inserted, delivery device 10 (or another suitable secondary device) may be inserted through sheath 110B. As they pass through sheath 110B, the portions of delivery device 10 that have a diameter larger than the inner diameter of the sheath in the reduced diameter configuration, such as distal sheath 24, may force ribs 116B and 117B to move away from one another. As seen in Fig.5D, delivery device 10 may apply forces directed radially outward from longitudinal axis L_{B}, such that the free end of one first rib 116B moves away from the free end of an adjacent second rib 117B, causing an increase in the size of gap 118B. Even as sheath 110B transitions to an expanded diameter configuration, such as that shown in Fig. 5D, the covering layer or layers maintain a fluid tight seal despite the increase in size of gap 118B. Because only certain portions of delivery device 10 may have a diameter larger than the interior diameter of sheath 11 0B in the reduced diameter configuration, the sheath may only expand locally as those larger size portions of the delivery device pass through the sheath. As delivery device 10 continues moving through sheath 11 0B, portions of the sheath that no longer overlie the relatively large portions of the delivery device may return to the reduced diameter configuration.

The procedure may be continued, for example by implanting a prosthetic heart valve, after which delivery device 10 may be removed from the patient. As delivery device 10 is retracted through sheath 110B, portions of the sheath will temporarily transition to the expanded diameter configuration to accommodate the relatively large portions of the delivery device, and then transition back to the reduced diameter configuration once the delivery device clears that portion of the sheath. After delivery device 10 has been completely removed from sheath 110B and the patient, the entire sheath will be in the reduced diameter configuration because no radially outward forces will be applied to the sheath from the interior thereof. Sheath 110B may be removed from the patient while in the reduced diameter configuration to minimize trauma to the patient's vasculature.

Although in the illustrated embodiment, each first rib 116B is aligned at the same longitudinal position on spine 115B with a corresponding second rib 117B, this does not need to be the case. For example, in other embodiments, first ribs 116B and second ribs 117B may be at staggered positions with respect to one another along the length of spine 115B. Further, each pair of ribs including a first rib 116B and a second rib 117B may extend along a plane that is orthogonal to longitudinal axis L_{B}, but in other embodiments they may extend along a plane that is oblique to the longitudinal axis. It should further be understood that, although the configuration of frame 111B in Fig. 5C is referred to as the reduced diameter configuration, the interior diameter of sheath 110B may decrease somewhat from that shown in Fig. 5C. For example, if radially inward forces are applied to frame 111B in the reduced diameter configuration, such as when sheath 110B is inserted through a constricted opening at the access site or a constricted portion of the patient's vasculature, pairs of adjacent first ribs 116B and second ribs 117B may move toward one another, decreasing the size of or eliminating gap 118B. Such movement of first ribs 116B and second ribs 117B to reduce or eliminate gap 118B may be helpful in navigating the vasculature, particularly where the vasculature is especially tortuous.

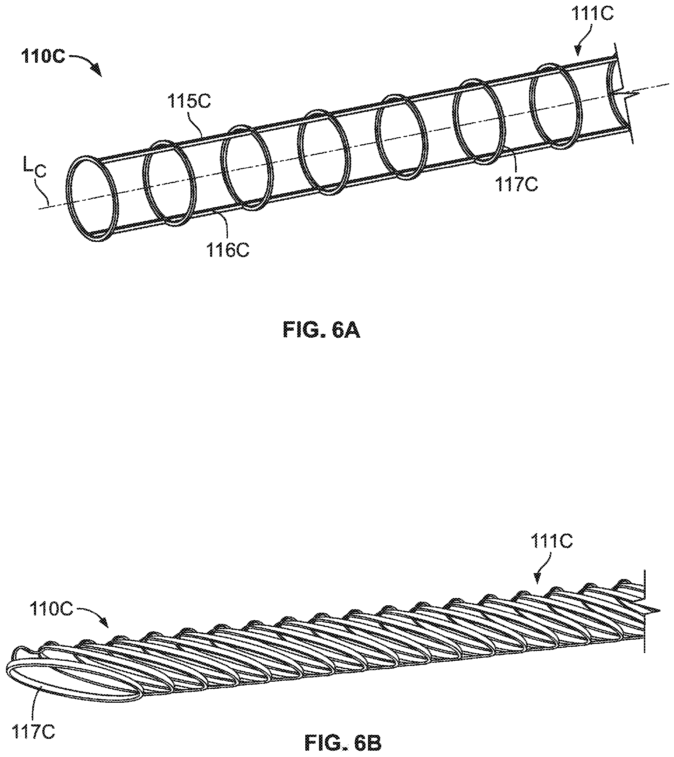

Fig. 6A is a perspective view of a portion of a further embodiment of an expandable distal sheath 110C which may form a part of introducer 100. Sheath 110C may include a frame 111C comprising a first support 115C and a second support 116C extending the entire length of the sheath, with a plurality of closed rings 117C spaced apart from one another along the frame. One or both supports 115C and 116C are preferably rigid enough so that a pushing force exerted on a proximal end of the support is transmitted to the distal end of the support. Supports 115C, 116C may have any suitable cross-section, including circular, square, or rectangular, for example. In other embodiments, each support 115C, 116C may have a curved shape similar to that described above in connection with the spine 115B of frame 111B. It may be preferable for supports 115C, 116C to have a square or rectangular cross-section to help mitigate twisting of the supports about their longitudinal axes during use. In some embodiments, supports 115C, 116C may be formed of a metal, a metal alloy such as Nitinol, or a stiff polymer. Rings 117C are each preferably similar or identical to one another and are formed of a flexible metal, polymer, elastomer, or combinations thereof. Rings 117C may have cross-sections that are rectangular, square, circular, or some other shape.

Still referring to Fig. 6A, each ring 117C preferably is circular in the absence of applied forces. Each ring 117C is coupled to first support 115C and to second support 116C at diametrically opposed portions of the ring. Any suitable method of attachment may be used to attach each ring 117C to supports 115C, 116C, so long as the points of attachment of the rings to the respective supports remain substantially stationary. In other words, rings 117C may be fixedly attached to supports 115C, 116C, for example by welding, adhesives, and the like, or the rings may be hingedly or pivotably attached to the corresponding supports. Each ring 117C may be connected to supports 115C, 116C on the inner diameter of the ring or on the outer diameter of the ring.

In order to maintain a fluid-tight seal between the space inside frame 111C and the space outside the frame, an expandable outer layer of material, similar to layer 112A of sheath 110A, may cover the exterior of the frame. For example, a thin cylindrical covering may extend around the exterior of frame 111C, including around the spacing between adjacent rings 117C. The material covering the exterior surfaces of frame 111C is preferably thin and able to expand and contract as the shape of the frame changes, while maintaining a fluid tight seal between the inside of the frame and the outside of the frame. Similar to the material forming layer 112A of sheath 110A, the material covering the exterior of frame 111C may be a polyimide material with a hydrophilic coating to help reduce friction. The interior surface of frame 111C may be similarly covered with a polyimide material having a hydrophilic coating, such that the frame is effectively encapsulated by the coating or covering material. In other words, the layers of material, alone or in combination with frame 111C, may provide for a substantially cylindrical, fluid tight structure. However, as noted with other embodiments described herein, these materials are merely exemplary and other materials may be suitable for providing the desired functionality.

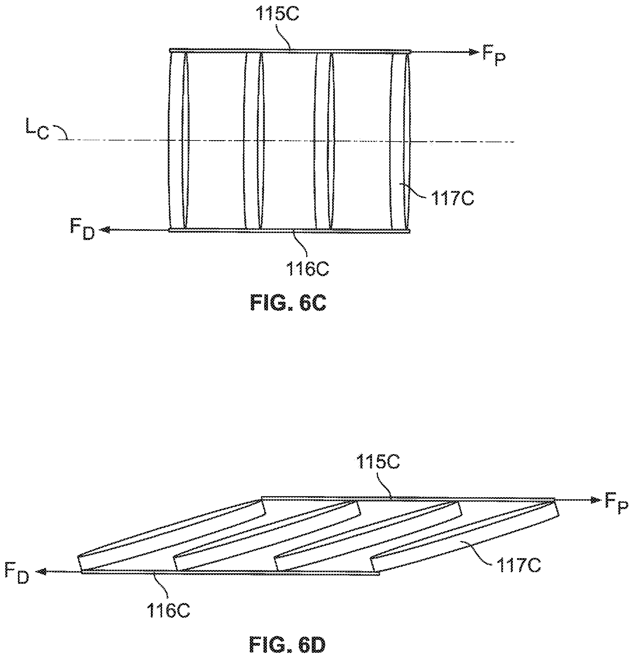

In the expanded diameter configuration shown in Figs. 6A and 6C, each ring 117C is substantially circular and defines a plane that is substantially orthogonal to the longitudinal axis L_{C} extending through sheath 110C. In order to transition sheath 110C from the expanded diameter configuration of Figs. 6A and 6C to the reduced diameter configuration of Figs.6B and 6D, a user may apply a proximally directed force F_{P} to first support 115C in a direction parallel to longitudinal axis L_{C} while second support 116C is held stationary, or a user may apply a distally directed force F_{D} to second support 116C in a direction parallel to the longitudinal axis while first support 115C is held stationary. As should be understood, the proximally directed force F_{P} and distally directed force F_{D} may be applied simultaneously to frame 111C to transition sheath 110C to the reduced diameter configuration. As first support 115C moves proximally with respect to second support 116C, rings 117C move in unison with one another and the planes defined by the rings transition to oblique orientations with respect to longitudinal axis L_{C}. Further, as sheath 110C begins to transition to the reduced diameter configuration, first support 115C moves closer to second support 116C while the supports remain substantially parallel to both one another and to longitudinal axis L_{C}. During this initial stage, rings 117C may remain substantially circular as the rings effectively pivot about their points of attachment supports 115C, 116C as the supports move closer together. However, as additional force is applied to one or both supports 115C, 116C while the supports remain spaced a distance from one another, the rings 117C may begin to stretch to an oval or elliptical shape. While rings 117C maintain an elliptical shape, the major axis X_{MAJOR} of the ellipse may bisect an imaginary line extending from first support 115C to second support 116C.

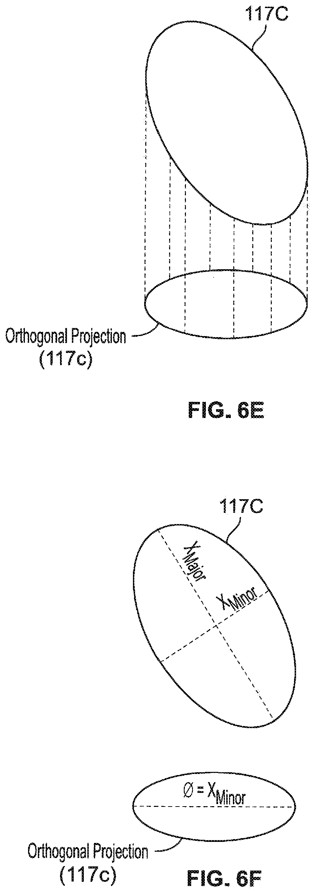

Fig. 6E illustrates the concept that when a ring 117C is stretched to an ellipse and oriented at an oblique angle relative to its central axis (i.e. longitudinal axis L_{C}), an orthogonal projection of the ellipse may form a circle. Fig. 6F illustrates that although ring 117C may be stretched to an ellipse having a major axis X_{MAJOR} and a minor axis X_{MINOR}, the orthogonal projection of the ring may be circular with a diameter equal to the length of the minor axis. A covering sheath with an inner diameter approximately equal to the length of minor axis X_{MlNOR} may be used to maintain sheath 110C in the reduced diameter configuration shown in Figs.6B and 6D. In an exemplary procedure, sheath 110C may be transitioned to the reduced diameter configuration and positioned within the covering sheath, and the assembly may then be inserted into the vasculature of the patient at the access site while the sheath is maintained in the reduced diameter configuration by the covering sheath. Once fully inserted, the covering sheath may be retracted, exposing sheath 110C. Depending on the angle at which rings 117C are tilted, the user may either pull first support 115C proximally while holding second support 116C stationary (or otherwise push the second support distally), or pull the second support proximally while holding the first support stationary (or otherwise push the first support distally), in order to transition sheath 110C to the expanded diameter configuration. In other embodiments, rings 117C may be shape-set so that, upon removal of the covering sheath, the rings tend to expand back to the expanded diameter configuration. In the expanded diameter configuration, each ring 117C is substantially circular with a diameter that is greater than minor axis X_{MINOR} but smaller than major axis X_{MAJOR}. Delivery device 10 (or another suitable secondary device), may then be passed through sheath 110C while the sheath is in the expanded diameter configuration. The procedure may be continued, for example by implanting a prosthetic heart valve, after which delivery device 10 may be removed from the patient while sheath 110C remains in the expanded diameter configuration. Prior to removing sheath 110C from the patient, it may be transitioned back to the reduced diameter configuration. This may be accomplished by manipulating supports 115C, 116C in a similar fashion to that described above, and then advancing the covering sheath back over sheath 110C to keep it in the reduced diameter configuration during removal from the patient. However, in some embodiments, sheath 110C may be compliant enough to remove from the patient without re-positioning the covering sheath over sheath 110C.

It should be understood that first support 115C and second support 116C need not both be rigid members in all embodiments. In some embodiments, either first support 115C or second support 116C could be non-rigid string-like members. In such an embodiment, the rigid support could be held stationary while the non-rigid support could be pulled proximally to cause rings 117C to change orientation. However, the non-rigid support in this embodiment may not be effective in transmitting a distal pushing force to change the orientation of rings 117C. Further, although rings 117C are described above as being elliptical in the reduced diameter configuration such that the ellipse orthogonally projects to a circle, such a configuration is not strictly necessary. Still further, it may be possible to achieve the desired change in diameter of sheath 110C with the use of a single rigid support, instead of the two supports described above.

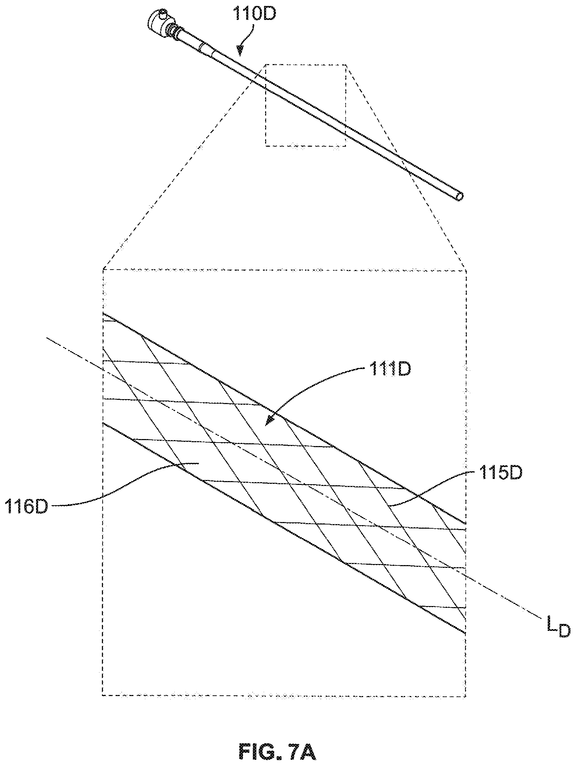

Fig. 7A is a perspective view of a portion of embodiment of an expandable distal sheath 110D which may form a part of introducer 100, the sheath being shown in an expanded diameter configuration. Sheath 110D may include a tubular or cylindrical frame 111D that includes a plurality of struts 115D forming cells 116D. In the illustrated embodiment, cells 116D are substantially diamond shaped. Frame 111D is preferably formed from a shape-memory metal alloy, such as Nitinol. In one embodiment, frame 111D may be laser cut from a continuous tube of Nitinol, and is preferably shape-set in the expanded configuration. In order to maintain a fluid-tight seal between the space inside frame 111D and the space outside the frame, an expandable layer of material may cover the frame, similar to layer 112A of sheath 110A. Extrusion, impregnation, or other processes may be used such that the material forms a substantially cylindrical tube, preferably with frame 111D embedded within the material so that a layer of material covers both the inside and outside of the frame. The layer is preferably formed of a highly elastic material to allow for expansion and contraction of frame 111D, described in greater detail below. In one embodiment, the material is a polyimide material with a hydrophilic coating.

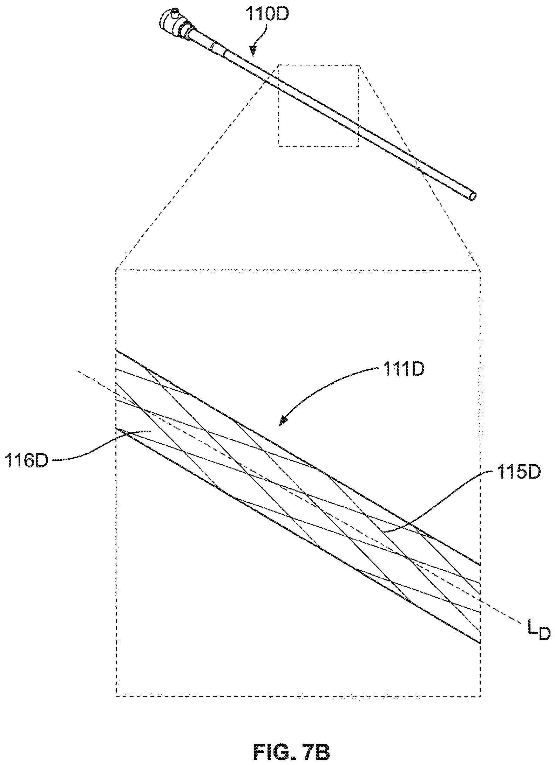

Frame 111D may have a positive Poisson ratio. In other words, if frame 111D is compressed along its longitudinal axis L_{D}, the frame will expand in a direction orthogonal to the longitudinal axis. This condition is illustrated in Fig. 7A and may be referred to as the expanded diameter configuration. Conversely, if frame 111D is extended along longitudinal axis L_{D}, the frame will compress in a direction orthogonal to the longitudinal axis. This condition is illustrated in Fig. 7B and may be referred to as the reduced diameter configuration. As frame 111D transitions from the expanded diameter condition to the reduced diameter configuration, two opposite apices of each cell 116D move closer to one another while the other two opposite apices move apart from one another.

Preferably, frame 111D is shape-set in the expanded diameter configuration shown in Fig.7A such that, in the absence of applied forces, sheath 110D has an inner diameter sufficiently large for delivery device 10 or other secondary devices to pass through the sheath. In this embodiment, sheath 110D may be transitioned to the reduced diameter configuration by compressing the sheath within a covering sheath having an interior diameter that is smaller than the exterior diameter of sheath 110D in the expanded diameter configuration. The covering sheath has sufficient hoop strength to maintain sheath 110D in the reduced diameter configuration. While the covering sheath maintains sheath 110D in the reduced diameter configuration, the assembly may be inserted into the vasculature of the patient at the access site. Once fully inserted, the covering sheath may be retracted proximally, exposing sheath 110D and allowing frame 111D and sheath 110D to expand to the expanded diameter configuration. As described above, this expansion may result from the shape-memory material of frame 111D returning to its pre-set shape. Delivery device 10 (or another suitable secondary device) may be passed through sheath 110D while the sheath is in the expanded diameter configuration. The procedure may be continued, for example by implanting a prosthetic heart valve, and delivery device 10 may be removed from the patient while sheath 110D remains in the expanded diameter configuration. Prior to removing sheath 110D from the patient, it may be transitioned back to the reduced diameter configuration. In one example, the covering sheath may be advanced over sheath 110D to transition it to the reduced diameter configuration, at which point sheath 110D may be removed from the patient while the covering sheath remains over it.

In other embodiments, sheath 110D may be actively expanded instead of relying on passive expansion as described above. For example, a substantially rigid member (not shown) may be attached to a distal end portion of sheath 110D. Sheath 110D may be inserted into the vasculature of the patient while in the reduced diameter configuration. Once sheath 110D is inserted to a desired position, the user may hold the rigid member stationary while applying a distal force to the proximal end of the sheath. The distal force will cause the proximal end of sheath 110D to move closer to the distal end of the sheath, which is being held stationary via the rigid member. As a result, the diameter of sheath 110D will expand as the distal force is applied. In this embodiment, frame 111D may not need to be formed of a shape-memory material, and it may even be preferable that the frame not be formed of a shape memory material so that sheath 110D does not tend to transition back to the reduced diameter configuration if the applied forces are removed. Once the procedure has been completed, the rigid member may be held stationary once more while a proximal force is applied to the proximal end of sheath 110D to collapse the sheath to the reduced diameter configuration, at which point the sheath may be removed from the patient. In this active expansion/contraction configuration, a covering sheath similar to that described above in connection with the passive expansion configuration may not be necessary. In still other embodiments, frame 111D may be formed of a shape memory material and shape-set to the reduced diameter configuration instead of the expanded diameter configuration. In such an embodiment, expansion may occur when the additional device, such as delivery device 10, passes through sheath 110D and forces frame 111D to expand.

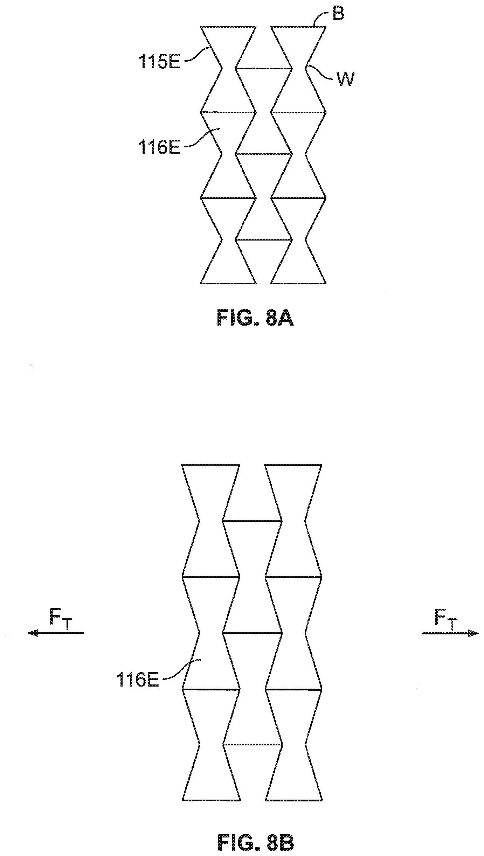

Whereas frame 111D of sheath 110D includes a structure that has a positive Poisson ratio, an alternate embodiment may include a structure that has a negative Poisson ratio, otherwise known as an auxetic structure. The concept of an auxetic structure is first briefly explained in connection with Figs.8A-B. The particular auxetic structure of Figs.8A-B includes a plurality of repeating cells 116E having an hourglass shape, each cell including two bases B of constant length coupled to one another by four struts 115E that form a narrower waist W between the bases. Cells 116E of the auxetic structure may have a shape similar to that shown in Fig.8A in the absence of applied forces. Referring to Fig. 8B, if a tensile force F_{T} is applied to the auxetic structure in directions parallel to bases B, which may be referred to as the longitudinal direction, the struts 115A forming the waist W of each cell 116E change angles with respect to the corresponding bases B. In particular, as tensile forces F_{T} are applied in the longitudinal direction, the angle between struts 115E and a corresponding base B approach a right angle. The distance between the two bases B of a cell 116E is at a maximum when the struts 115E are all perpendicular to the bases B (although this positioning may not be achievable in operation). Thus, as tensile forces F_{T} are applied and the struts 115E approach perpendicularity with the bases B, the auxetic structure expands in the direction perpendicular to the longitudinal direction. Conversely, if a compressive force is applied in the longitudinal direction, the auxetic structure collapses in the direction perpendicular to the longitudinal direction. This result is substantially opposite that achieved with the diamond-shaped cells of frame 111D, where tensile forces in one direction result in the structure collapsing in a direction perpendicular to that direction, and compressive forces in one direction result in expansion in the perpendicular direction.

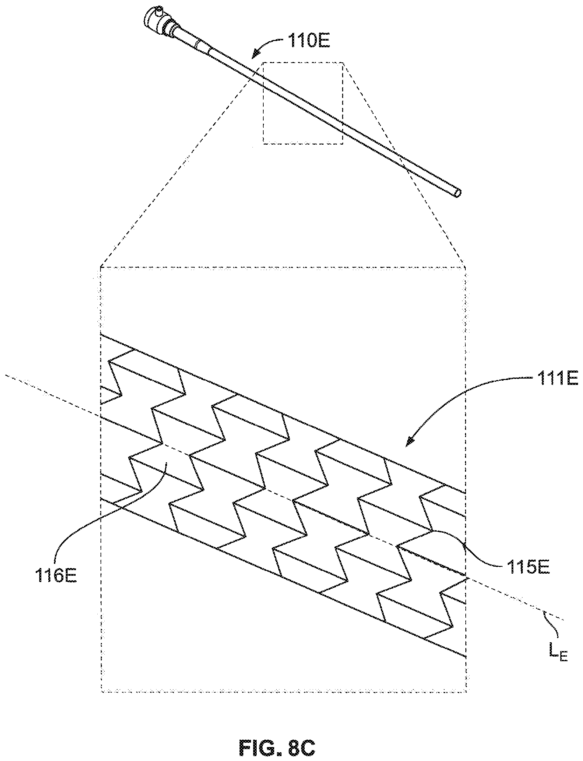

Fig. 8C is a perspective view of a portion of an expandable distal sheath 110E that may form a part of introducer 100, and is shown in a reduced diameter configuration. Sheath 110E may include a tubular or cylindrical frame 111E that includes the auxetic structure of Figs.8A-B. In particular, frame 111E may include a plurality of hourglass shaped cells 116E arranged to form a cylindrical configuration, with the bases B of each cell aligned substantially parallel to the longitudinal axis L_{E} of the frame. Frame 111E is preferably formed from a shape-memory metal alloy, such as Nitinol. In one embodiment, frame 111E may be laser cut from a continuous tube of Nitinol, and is preferably shape-set in the expanded configuration of Fig. 8B. In order to maintain a fluid-tight seal between the space inside frame 111E and the space outside the frame, an expandable layer of material may cover the frame, similar to layer 112A of sheath 110A. Extrusion, impregnation, or other processes may be used such that the material forms a substantially cylindrical tube, preferably with frame 111E embedded within the material so that a layer of material covers both the inside and the outside of the frame. The layer is preferably formed of a highly elastic material to allow for expansion and contraction of frame 111E. In one embodiment, the material is a polyimide material with a hydrophilic coating.

As noted above, frame 111E is preferably shape-set in the expanded diameter configuration shown in Fig. 8B. With this configuration, in the absence of applied forces, sheath 110E preferably has an inner diameter sufficiently large for delivery device 10 or other secondary devices to pass through the sheath. In this embodiment, sheath 110E may be transitioned to the reduced diameter configuration by compressing the sheath within a covering sheath having an interior diameter that is smaller than the exterior diameter of sheath 110E in the expanded diameter configuration. The covering sheath has sufficient hoop strength to maintain sheath 110E in the reduced diameter configuration. While the covering sheath maintains sheath 110E in the reduced diameter configuration, the assembly may be inserted into the vasculature of the patient at the access site. Once inserted to a desired position, the covering sheath may be retracted proximally, exposing sheath 110E and allowing frame 111E and sheath 110E to expand to the expanded diameter configuration. As described above, this expansion may result as the shape-memory material of frame 111E returns to its pre-set shape. Delivery device 10 (or another suitable secondary device) may be passed through sheath110E while the sheath is in the expanded diameter configuration. The procedure may be continued, for example by implanting a prosthetic heart valve, and delivery device 10 may be removed from the patient while sheath 110E remains in the expanded diameter configuration. Prior to removing sheath 110E from the patient, it may be transitioned back to the reduced diameter configuration. In one example, the covering sheath may be advanced over sheath 110E to transition it to the reduced diameter configuration, at which point sheath 110E may be removed from the patient while the covering sheath remains over it.

In other embodiments, sheath 110E may be actively expanded instead of relying on passive expansion as described above. For example, a substantially rigid member (not shown) may be attached to a distal end portion of sheath 110E. Sheath 110E may be inserted into the vasculature of the patient while in the reduced diameter configuration. Once sheath 110E is inserted to a desired position, the user may hold the rigid member stationary while applying a proximal force to the proximal end of the sheath. The resultant tensile force will cause the diameter of sheath 110E to expand, as explained in connection with Figs.8A-B. In this embodiment, frame 111E may not need to be formed of a shape-memory material, and it may even be preferable that the frame not be formed of a shape memory material so that sheath 110E does not tend to transition back to the reduced diameter configuration if the applied forces are removed. Once the procedure has been completed, the rigid member may be held stationary once more while a distal or compressive force is applied to the proximal end of sheath 110E to collapse the sheath to the reduced diameter configuration, at which point the sheath may be removed from the patient. In this active expansion/contraction configuration, a covering sheath similar to that described above in connection with the passive expansion configuration may not be necessary.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the scope of the present invention as defined by the appended claims.

## Claims

1. An introducer (100) for providing access to a surgical site in a patient comprising:
a sheath (110A) extending from a proximal portion to a distal portion along a longitudinal axis (L_{A}), the sheath including a coil (111A) having a plurality of turns (T1, T2, T3) spiraling about the longitudinal axis,
wherein the coil has a reduced diameter configuration in which portions of the coil forming a multiplicity of the turns are spaced a first distance from the longitudinal axis in a radial direction orthogonal to the longitudinal axis, and an expanded diameter configuration in which the portions of the coil forming the multiplicity of the turns are spaced a second distance from the longitudinal axis in the radial direction, the second distance greater than the first distance
**characterized in that** each turn includes a body (115A) that tapers in a first direction and transitions into a flange (117A) that is wider than the narrowest portion of the taper, and a recess (118A) positioned opposite the flange, each flange configured to passively sit within a portion of the recess of a next adjacent turn.

2. The introducer (100) of claim 1, wherein the sheath includes a substantially cylindrical material (112A) at least partially covering the coil, (111A) the cylindrical material being expandable with the coil.

3. The introducer (100) of claim 1, wherein the coil (111A) includes a proximal end and a distal end, the proximal end being spaced apart a first distance from the distal end in a longitudinal direction parallel to the longitudinal axis (L_{A}) when in the reduced diameter configuration, the proximal end of the coil being spaced apart a second distance from the distal end of the coil when in the expanded diameter configuration, the first distance being greater than the second distance.

4. The introducer 100 of claim 1, wherein the coil (111A) includes an aperture (116A) extending along the plurality of turns (T1, T2, T3) of the coil, and a pull-wire is positioned extending through the aperture along the plurality of turns.

5. The introducer (100) of claim 4, wherein the aperture (116A) is positioned within the body (115A) of each turn (T1, T2, T3).

6. The introducer (100) of claim 1, wherein the recess (118A) is formed by sidewalls that extend to form two pointed prongs.

## Patentansprüche

1. Einführvorrichtung (100) zur Schaffung eines Zugangs zu einer chirurgischen Stelle bei einem Patienten, umfassend:
eine Schleuse (110A), die sich von einem proximalen Abschnitt zu einem distalen Abschnitt entlang einer Längsachse (L_{A}) erstreckt, wobei die Schleuse eine Spule (111A) einschließt, die eine Vielzahl von Windungen (T1, T2, T3) aufweist, die sich spiralförmig um die Längsachse winden,
wobei die Spule eine Konfiguration mit reduziertem Durchmesser aufweist, bei der Abschnitte der Spule, die eine Vielzahl von Windungen bilden, in einer radialen Richtung orthogonal zur Längsachse einen ersten Abstand von der Längsachse aufweisen, und eine Konfiguration mit erweitertem Durchmesser, bei der die Abschnitte der Spule, die die Vielzahl von Windungen bilden, in der radialen Richtung einen zweiten Abstand von der Längsachse aufweisen, wobei der zweite Abstand größer als der erste Abstand ist,
**dadurch gekennzeichnet, dass** jede Windung einen Körper (115A) einschließt, der sich in einer ersten Richtung verjüngt und in einen Flansch (117A) übergeht, der breiter ist als der schmalste Abschnitt der Verjüngung, und eine Aussparung (118A), die gegenüber dem Flansch angeordnet ist, wobei jeder Flansch so konfiguriert ist, dass er passiv in einem Abschnitt der Aussparung einer nächsten benachbarten Windung sitzt.

2. Einführvorrichtung (100) nach Anspruch 1, wobei die Schleuse ein im Wesentlichen zylindrisches Material (112A) einschließt, das die Spule (111A) zumindest teilweise bedeckt, wobei das zylindrische Material mit der Spule expandierbar ist.

3. Einführvorrichtung (100) nach Anspruch 1,
wobei die Spule (111A) ein proximales Ende und ein distales Ende einschließt, wobei das proximale Ende in der Konfiguration mit reduziertem Durchmesser in einer Längsrichtung parallel zur Längsachse (L_{A}) um einen ersten Abstand vom distalen Ende beabstandet ist,
das proximale Ende der Spule in einem zweiten Abstand vom distalen Ende der Spule angeordnet ist, wenn sie sich in der Konfiguration mit erweitertem Durchmesser befindet,
der erste Abstand größer ist als der zweite Abstand.

4. Einführvorrichtung 100 nach Anspruch 1, wobei die Spule (111A) eine Öffnung (116A) einschließt, die sich entlang der Vielzahl von Windungen (T1, T2, T3) der Spule erstreckt, und ein Zugdraht angeordnet ist, der sich durch die Öffnung entlang der Vielzahl von Windungen erstreckt.

5. Einführvorrichtung (100) nach Anspruch 4, wobei die Öffnung (116A) innerhalb des Körpers (115A) jeder Windung (T1, T2, T3) angeordnet ist.

6. Einführvorrichtung (100) nach Anspruch 1, wobei die Aussparung (118A) von Seitenwänden gebildet ist, die sich so erstrecken, dass sie zwei spitze Zacken bilden.

## Revendications

1. Dispositif d'introduction (100) destiné à fournir un accès à un site chirurgical chez un patient comprenant :
une gaine (110A) s'étendant depuis une portion proximale vers une portion distale le long d'un axe longitudinal (L_{A}), la gaine incluant une bobine (111A) présentant une pluralité de tours (T1, T2, T3) en spirale autour de l'axe longitudinal,
dans lequel la bobine présente une configuration de diamètre réduit où des portions de la bobine formant une multiplicité de tours sont espacées d'une première distance par rapport à l'axe longitudinal dans une direction radiale orthogonale à l'axe longitudinal et une configuration de diamètre étendu où les portions de la bobine formant la multiplicité de tours sont espacées d'une seconde distance par rapport à l'axe longitudinal dans la direction radiale, la seconde distance étant supérieure à la première distance,
**caractérisé en ce que** chaque tour inclut un corps (115A) qui devient plus étroit dans une première direction et fait une transition vers une bride (117A) qui est plus large que la portion la plus étroite du cône et un évidement (118A) positionné de manière opposée à la bride, chaque bride étant configurée pour se positionner de manière passive à l'intérieur d'une portion de l'évidement d'un trou adjacent suivant.

2. Dispositif d'introduction (100) selon la revendication 1, dans lequel la gaine inclut un matériau sensiblement cylindrique (112A) recouvrant au moins partiellement la bobine (111A), le matériau cylindrique étant déployable avec la bobine.

3. Dispositif d'introduction (100) selon la revendication 1,
dans lequel la bobine (111A) inclut une extrémité proximale et une extrémité distale, l'extrémité proximale étant espacée d'une première distance par rapport à l'extrémité distale dans une direction longitudinale parallèle à l'axe longitudinal (L_{A}) dans la configuration de diamètre réduit,
l'extrémité proximale de la bobine étant espacée d'une seconde distance par rapport à l'extrémité distale de la bobine dans la configuration de diamètre étendu,
la première distance étant supérieure à la seconde distance.

4. Dispositif d'introduction (100) selon la revendication 1, dans lequel la bobine (111A) inclut une ouverture (116A) s'étendant le long de la pluralité de tours (T1, T2, T3) de la bobine et un fil de traction est positionné s'étendant à travers l'ouverture le long de la pluralité de tours.

5. Dispositif d'introduction (100) selon la revendication 4, dans lequel l'ouverture (116A) est positionnée à l'intérieur du corps (115A) de chaque tour (T1, T2, T3).

6. Dispositif d'introduction (100) selon la revendication 1, dans lequel l'évidement (118A) est formé par des parois latérales qui s'étendent pour former deux dents pointues.
